# EUROPEAN PATENT APPLICATION

(11) **EP 4 487 964 A1**
(43) Date of publication of application: **08.01.2025**
(21) Application number: 23795227.0
(22) Date of filing: 21.04.2023
(51) Int. Cl.: B05B 17/06

(54) **ULTRASONIC ATOMIZATION APPARATUS**

(30) Priority: 27.04.2022 CN 202210457228
(71) Applicant: Shenzhen First Union Technology Co., Ltd., Shenzhen, Guangdong 518000 (CN)
(72) Inventor: LI, Xinjun, Shenzhen, Guangdong 518000 (CN); XU, Zhongli, Shenzhen, Guangdong 518000 (CN); LI, Yonghai, Shenzhen, Guangdong 518000 (CN)
(74) Representative: Ran, Handong
(86) International application number: PCT/CN2023/089808
(87) International publication number: WO 2023/207794

(57) **Abstract**

Disclosed in the present application is an ultrasonic atomization apparatus, comprising: a liquid storage cavity for storing a liquid matrix; an ultrasonic atomization sheet, which is in communication with the liquid storage cavity and generates oscillation to atomize the liquid matrix; a power source; and a driving control circuit, which comprises a controller, a driving circuit and an impedance matching circuit, wherein the impedance matching circuit comprises a capacitor branch and an inductive branch; the capacitor branch is connected to the ultrasonic atomization sheet in series to form a first circuit, and the inductive branch is connected to the first circuit to form a second circuit; and the driving circuit is respectively connected to the controller, the power source and the second circuit, and is configured to output, on the basis of the control of the controller, a driving voltage used for driving the ultrasonic atomization sheet. In this way, the working efficiency of an ultrasonic atomization sheet can be effectively improved.

## Description

### CROSS-REFERENCE TO RELATED APPLICATIONS

This application claims priority to Chinese Patent Application No. 202210457228.8, filed with the China National Intellectual Property Administration on April 27, 2022 and entitled "ULTRASONIC ATOMIZATION APPARATUS", which is incorporated herein by reference in its entirety.

### TECHNICAL FIELD

This application relates to the field of atomizer technologies, and in particular, to an ultrasonic atomization apparatus.

### BACKGROUND

An ultrasonic atomization apparatus is an apparatus that implements an atomization function by using an ultrasonic atomization technology.

Currently, during use of the ultrasonic atomization apparatus, to enable an ultrasonic atomization sheet in the ultrasonic atomization apparatus to have high operating efficiency, the ultrasonic atomization sheet is usually controlled to operate near a series resonant point of the ultrasonic atomization sheet. In this case, the ultrasonic atomization sheet is in a capacitive state to the outside.

However, when a power supply supplies power to the ultrasonic atomization sheet in the capacitive state, there is a large phase difference between an operating voltage and an operating current on the ultrasonic atomization sheet, resulting in low operating efficiency of the ultrasonic atomization sheet.

### SUMMARY

Embodiments of this application aim to provide an ultrasonic atomization apparatus, to effectively improve operating efficiency of an ultrasonic atomization sheet.

According to a first aspect, this application provides an ultrasonic atomization apparatus, including:
a liquid storage cavity, configured to store a liquid substrate;
an ultrasonic atomization sheet, where the ultrasonic atomization sheet is in liquid communication with the liquid storage cavity, and the ultrasonic atomization sheet is configured to generate oscillation to atomize the liquid substrate; and
a power supply and a driving control circuit, where
the driving control circuit includes a controller, a driving circuit, and an impedance matching circuit, where
the impedance matching circuit includes a capacitance branch and an inductance branch; the capacitance branch and the ultrasonic atomization sheet are connected in series to form a first circuit, and the inductance branch is connected to the first circuit to form a second circuit; and
the driving circuit is separately connected to the controller, the power supply, and the second circuit, and the driving circuit is configured to output, based on control of the controller, a driving voltage used to drive the ultrasonic atomization sheet.

According to a second aspect, this application provides an ultrasonic atomization apparatus, including:
a liquid storage cavity, configured to store a liquid substrate;
an ultrasonic atomization sheet, where the ultrasonic atomization sheet is in liquid communication with the liquid storage cavity, and the ultrasonic atomization sheet is configured to generate oscillation to atomize the liquid substrate; and
a power supply and a driving control circuit, where
the driving control circuit includes a controller, a dual-switch boost driving circuit, and an impedance matching circuit, where
the impedance matching circuit includes a capacitance branch and an inductance branch; the capacitance branch and the ultrasonic atomization sheet are connected in series to form a first circuit, and the inductance branch is connected to the first circuit to form a second circuit; and
the dual-switch boost driving circuit is separately connected to the controller, the power supply, and the second circuit, and the dual-switch boost driving circuit is configured to alternately output, based on control of the controller, a first driving voltage and a second driving voltage that are used to drive the ultrasonic atomization sheet.

According to a third aspect, this application provides an ultrasonic atomization apparatus, including:
a power supply mechanism and an ultrasonic atomizer, where
the ultrasonic atomizer includes:
   a first housing, where a liquid storage cavity is formed in the first housing and is configured to store a liquid substrate;
   an ultrasonic atomization sheet disposed in the first housing, where the ultrasonic atomization sheet is in liquid communication with the liquid storage cavity, and the ultrasonic atomization sheet is configured to generate oscillation when receiving a driving voltage, to atomize the liquid substrate; and
   the power supply mechanism includes:
      a second housing;
      a power supply and a driving control circuit that are disposed in the second housing, where the driving control circuit includes a controller, a driving circuit, and an impedance matching circuit, the driving circuit is separately connected to the power supply and the controller, and the impedance matching circuit includes a capacitance branch and an inductance branch;
      when the first housing is coupled to the second housing, the capacitance branch and the ultrasonic atomization sheet are connected in series to form a first circuit, the inductance branch is connected to the first circuit to form a second circuit, and the driving circuit is connected to the second circuit; and the driving circuit is configured to output, based on control of the controller, a driving voltage used to drive the ultrasonic atomization sheet.

An ultrasonic atomization apparatus provided in the embodiments of this application includes a liquid storage cavity, an ultrasonic atomization sheet, a power supply, a controller, a driving circuit, and an impedance matching circuit. The impedance matching circuit includes a capacitance branch and an inductance branch. The capacitance branch and the ultrasonic atomization sheet are connected in series to form a first circuit. The inductance branch is connected to the first circuit to form a second circuit. Through disposing of the first circuit and the second circuit, when a change range of an equivalent capacitance facing the ultrasonic atomization sheet is relatively large, an overall impedance feature of the second circuit is still stably maintained in an inductive state. Further, a phase difference between an operating current and an operating voltage on the ultrasonic atomization sheet can be reduced, so that a useful power on the ultrasonic atomization sheet is increased, and a heating power on the ultrasonic atomization sheet is reduced, to improve operating efficiency of the ultrasonic atomization sheet, and also help reduce a heating temperature of the ultrasonic atomization sheet.

### BRIEF DESCRIPTION OF THE DRAWINGS

One or more embodiments are exemplarily described with reference to the corresponding figures in the accompanying drawings, and the descriptions are not to be construed as limiting the embodiments. Elements in the accompanying drawings that have same reference numerals are represented as similar elements, and unless otherwise particularly stated, the figures in the accompanying drawings are not drawn to scale.
FIG. 1 is a schematic diagram of a structure of an ultrasonic atomization apparatus according to an embodiment of this application;
FIG. 2 is a schematic diagram of a structure of an ultrasonic atomization apparatus according to another embodiment of this application;
FIG. 3 is a schematic diagram of a structure of a driving control circuit according to an embodiment of this application;
FIG. 4 is a schematic diagram of a circuit structure of a driving control circuit according to an embodiment of this application;
FIG. 5 is a schematic diagram of an operating voltage and an operating current that are applied to an ultrasonic atomization sheet when a capacitance branch and an inductance branch are not added according to an embodiment of this application;
FIG. 6 is a schematic diagram of an operating voltage and an operating current that are applied to an ultrasonic atomization sheet when a capacitance branch and an inductance branch are added according to an embodiment of this application;
FIG. 7 is a schematic diagram of a circuit structure of a driving control circuit according to another embodiment of this application;
FIG. 8 is a schematic diagram of a structure of a driving circuit according to an embodiment of this application; and
FIG. 9 is a schematic diagram of a structure of a driving circuit according to another embodiment of this application.

### DETAILED DESCRIPTION

To make the objectives, technical solutions, and advantages of embodiments of this application clearer, the following clearly and completely describes the technical solutions in embodiments of this application with reference to accompanying drawings in embodiments of this application. It is clear that the described embodiments are merely some rather than all of embodiments of this application. All other embodiments obtained by a person of ordinary skill in the art based on embodiments of this application without making creative efforts shall fall within the protection scope of this application.

Embodiments of this application provide an ultrasonic atomization apparatus. An impedance matching circuit connected to an ultrasonic atomization sheet is disposed in the ultrasonic atomization apparatus, where the impedance matching circuit includes a capacitance branch connected in series with the ultrasonic atomization sheet, and the capacitance branch and the ultrasonic atomization sheet form a first circuit; and the impedance matching circuit further includes an inductance branch connected to the first circuit, where the inductance branch and the first circuit form a second circuit. Through a design of the impedance matching circuit, when an equivalent capacitance facing the ultrasonic atomization sheet changes greatly, a capacitance value of the overall equivalent capacitance of the first circuit can be maintained within a specific range, so that an overall impedance feature of the second circuit can be stably maintained as inductive. When a power supply supplies power to the ultrasonic atomization sheet in an inductive state, a same phase or a relatively small phase difference may be maintained between an operating current and an operating voltage on the ultrasonic atomization sheet, so that a useful power on the ultrasonic atomization sheet is increased, and a heating power of the ultrasonic atomization sheet is reduced. That is, a heating temperature of the ultrasonic atomization sheet is reduced, and the ultrasonic atomization sheet can be kept to operate near a series resonant frequency point as much as possible, to improve operating efficiency of the ultrasonic atomization sheet.

For a plurality of ultrasonic atomization sheets (for example, ultrasonic atomization sheets in a same batch), different ultrasonic atomization sheets may have different features, parameters, or the like, resulting in different equivalent capacitances of the different ultrasonic atomization sheets when the different ultrasonic atomization sheets operate near the resonant frequency point. In this case, a change range of the capacitance value of the overall equivalent capacitance of the first circuit can also be controlled within a small range by disposing the capacitance branch, and the overall impedance feature of the second circuit can also be switched to be inductive by disposing the inductance branch, to reduce the heating temperature of the ultrasonic atomization sheet. In other words, during application of the plurality of ultrasonic atomization sheets, the capacitance branch and the inductance branch can be kept unchanged. That is, a problem of inconsistent atomization performance caused by differences in individual performance of the ultrasonic atomization sheets is resolved, and production efficiency of the ultrasonic atomization apparatus is also improved.

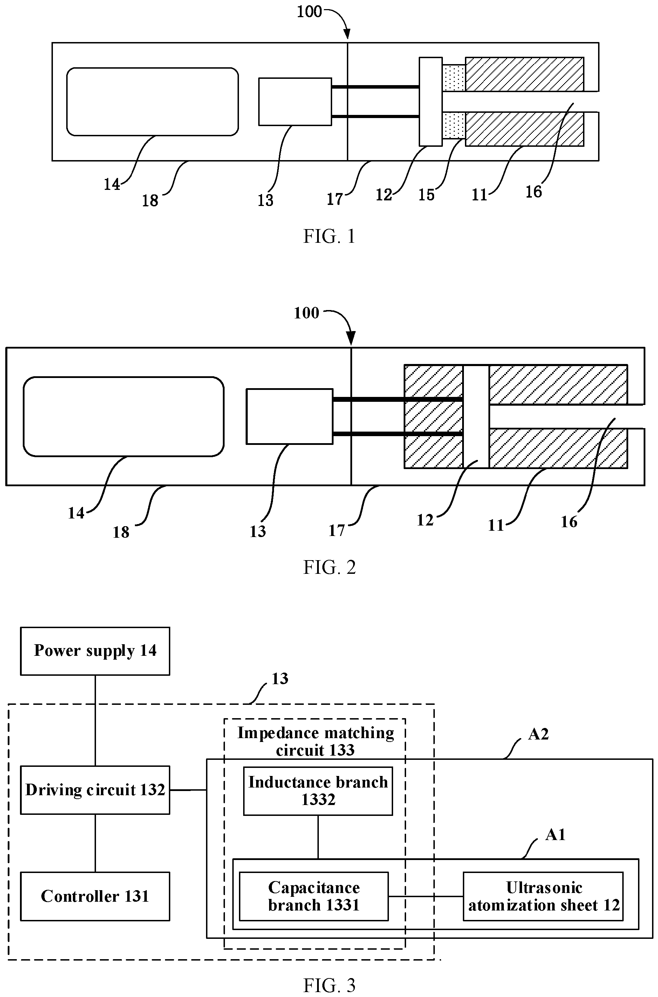

FIG. 1 is a schematic diagram of a structure of an ultrasonic atomization apparatus according to an embodiment of this application. As shown in FIG. 1, the ultrasonic atomization apparatus 100 includes a liquid storage cavity 11, an ultrasonic atomization sheet 12, a driving control circuit 13, and a power supply 14.

The liquid storage cavity 11 is configured to store a liquid substrate. The liquid substrate may include different substances according to different use scenarios. For example, in the field of e-cigarette atomization, the liquid substrate may include nicotine and/or an aroma and/or an aerosol-generation substance (for example, glycerol). For another example, in the field of medical atomization, solutions such as drugs and/or stroke-physiological saline solution for disease treatment or health benefits may be included.

The ultrasonic atomization sheet 12 is in fluid communication with the liquid storage cavity 11. The ultrasonic atomization sheet 12 may be directly disposed in the liquid storage cavity 11, or an atomization cavity in which the ultrasonic atomization sheet 12 is located is in direct communication with the liquid storage cavity 11, or liquid transmission is performed between the ultrasonic atomization sheet 12 and the liquid storage cavity 11 through a liquid absorbing medium. The ultrasonic atomization sheet 12 is configured to generate oscillation to atomize the liquid substrate. That is, the liquid substrate transferred to or near the ultrasonic atomization sheet 12 is atomized into an aerosol through vibration. Specifically, during use, the ultrasonic atomization sheet 12 breaks up the liquid substrate through high-frequency vibration (preferably, a vibration frequency is in a range of 1.7 MHz to 4.0 MHz, which exceeds a human hearing range and belongs to an ultrasonic frequency band) to generate an aerosol in which particles are naturally suspended.

The driving control circuit 13 is electrically connected to the ultrasonic atomization sheet 12. The driving control circuit 13 is configured to provide a driving voltage and a driving current for the ultrasonic atomization sheet 12 according to the power supply 14. In an implementation, the driving control circuit 13 may be disposed on a printed circuit board (PCB).

The power supply 14 is configured to supply power. In an implementation, the power supply 14 is a battery. The battery may be a lithium-ion battery, a lithium metal battery, a lead-acid battery, a nickel-cadmium battery, a nickel-metal hydride battery, a lithium-sulfur battery, a lithium-air battery, a sodium-ion battery, or the like. This is not limited herein. In terms of scale, the battery in the embodiments of this application may be a battery cell, or may be a battery module including a plurality of battery cells connected in series and/or connected in parallel. This is not limited herein. Certainly, in other embodiments, the battery may alternatively include more or fewer components, or have different component configurations. This is not limited in this embodiment of this application.

In an embodiment, the ultrasonic atomization apparatus 100 further includes a liquid transfer medium 15 and an air outlet channel 16.

The liquid transfer element 15 is configured to transfer the liquid substrate between the liquid storage cavity 11 and the ultrasonic atomization sheet 12.

The air outlet channel 16 is configured to output inhalable vapor or an aerosol generated by the liquid substrate for inhalation by a user.

The ultrasonic atomization apparatus 100 may be integrated or assembled. In an implementation, when the ultrasonic atomization apparatus 100 is assembled, the ultrasonic atomization apparatus 100 further includes a power supply mechanism and an ultrasonic atomizer. The ultrasonic atomizer includes a first housing 17, and the power supply mechanism includes a second housing 18.

The first housing 17 is detachably connected to the second housing 18. In an embodiment, the first housing 17 may be detachably connected to the second housing 18 through a buckle structure, a magnetic structure, or the like. The first housing 17 and the second housing 18 jointly function to accommodate and protect other components. The liquid storage cavity 11, the ultrasonic atomization sheet 12, the liquid transfer element 15, and the air outlet channel 16 are disposed in the first housing 17, and the driving control circuit 13 and the power supply 14 are disposed in the second housing 18.

The first housing 17 is detachably aligned with the second housing 18 in a functional relationship. Various mechanisms may be used to connect the second housing 18 to the first housing 17, to generate a threaded engagement, a press-fit engagement, an interference fit, a magnetic engagement, or the like. In some implementations, when the first housing 17 and the second housing 18 are assembled and configured, the ultrasonic atomization apparatus 100 may be basically in a shape of a rod, a flat cylinder, a rod, a column, or the like.

The first housing 17 and the second housing 18 may be formed of any suitable material that is structurally sound. In some examples, the first housing 17 and the second housing 18 may be formed by a metal or an alloy such as stainless steel or aluminum. Other suitable materials include various plastics (for example, polycarbonate), metal-plating over plastic (metal-plating over plastic), ceramics, and the like.

It should be noted that, a hardware structure of the ultrasonic atomization apparatus 100 shown in FIG. 1 is only an example. In addition, the ultrasonic atomization apparatus 100 may have more or fewer components than those shown in the figure, or may combine two or more components, or may have a different component configuration. The components shown in the figure may be implemented in hardware including one or more signal processing circuits and/or application-specific integrated circuits, software, or a combination of hardware and software. For example, as shown in FIG. 2, the ultrasonic atomization sheet 12 may be disposed in the liquid storage cavity 11, so that the liquid transfer element 15 can be saved, to help reduce costs.

In addition, it may be understood that, the ultrasonic atomization apparatus 100 shown in FIG. 1 or FIG. 2 may be applied to a plurality of different scenarios and play different roles. This is not specifically limited in this embodiment of this application. For example, in an embodiment, the ultrasonic atomization apparatus 100 is applied to the medical field. In this case, the ultrasonic atomizer 100 may be a medical atomizer. The medical atomizer can achieve an auxiliary treatment effect by atomizing a medical liquid added inside the medical atomizer and enabling a patient to inhale. For another example, in another embodiment, the ultrasonic atomization apparatus 100 may alternatively be used as an electronic product, for example, an e-cigarette. The e-cigarette is an electronic product that turns a nicotine solution and the like into vapor in a means such as atomization, for a user to inhale.

FIG. 3 is a schematic diagram of a structure in which a driving control circuit 13 is separately connected to a power supply 14 and an ultrasonic atomization sheet 12 according to an embodiment of this application. As shown in FIG. 3, the driving control circuit 13 includes a controller 131, a driving circuit 132, and an impedance matching circuit 133. The impedance matching circuit 133 includes a capacitance branch 1331 and an inductance branch 1332, the capacitance branch 1331 and the ultrasonic atomization sheet 12 are connected in series to form a first circuit A1, and the inductance branch 1332 is connected to the first circuit A1 to form a second circuit A2. The driving circuit 132 is separately connected to the controller 131, the power supply 14, and the second circuit A2. The driving circuit 132 is configured to output, based on control of the controller 131, a driving voltage used to drive the ultrasonic atomization sheet 12.

The controller 131 may be a microcontroller unit (Microcontroller Unit, MCU), a digital signal processing (Digital Signal Processing, DSP) controller, or the like. The controller 131 is electrically connected to the driving circuit 132. The controller 131 may be configured to control at least one electronic element in the driving circuit 132. In another implementation, the driving circuit 132 may alternatively be a dual-switch boost driving circuit.

During application of the ultrasonic atomization sheet 12, to improve operating efficiency of the ultrasonic atomization sheet 12, the ultrasonic atomization sheet 12 needs to operate as much as possible near a series resonant frequency point of the ultrasonic atomization sheet 12. In this case, the ultrasonic atomization sheet 12 is in a capacitive state to the outside. However, the ultrasonic atomization sheet 12 in the capacitive state may have the following problems.
1. When the power supply 14 supplies energy to the ultrasonic atomization sheet 12 in the capacitive state, there is a large phase difference between an operating voltage and an operating current on the ultrasonic atomization sheet 12. In this way, a useful power obtained on the ultrasonic atomization sheet 12 is small. As a result, efficiency of the power supply 14 is very low, that is, an input power is large, and a power obtained by load for operating is small. In addition, a remaining power in the input power is converted into heat generated by the power supply 14 or heat generated by the ultrasonic atomization sheet 12. As a result, the ultrasonic atomization sheet 12 heats severely, that is, a heating temperature is excessively high.
2. The ultrasonic atomization sheet 12 has unstable parameters, and it is difficult to control the ultrasonic atomization sheet 12. For example, for ultrasonic atomization sheets 12 in a same batch, consistency of different ultrasonic atomization sheets 12 is poor. As a result, capacitance values of equivalent capacitances of different ultrasonic atomization sheets 12 during operating are different. In this case, for different ultrasonic atomization apparatuses 100, corresponding control parameters need to be set according to different ultrasonic atomization sheets 12, resulting in increased difficulty in controlling the ultrasonic atomization sheets 12 and poor stability of finished products. In addition, during operating of the ultrasonic atomization sheet 12, the equivalent capacitance of the ultrasonic atomization sheet 12 also changes accordingly, which also increases difficulty of controlling the ultrasonic atomization sheet 12.
3. It is difficult to effectively perform feedback control on the ultrasonic atomization sheet 12 through an input current. Because the ultrasonic atomization sheet 12 is in a capacitive state, an instantaneous current is sometimes abnormally large, and sometimes changes abruptly, which is very unstable. Control of the ultrasonic atomization sheet 12 is usually based on detection on the input current. In this case, difficulty in controlling a microprocessor is also great.

In this embodiment of this application, the capacitance branch 1331 and the inductance branch 1332 are added, so that a feature of an unstable equivalent capacitance of the ultrasonic atomization sheet 12 can be overcome, and an impedance feature of the second circuit A2 is effectively maintained as inductive, so that a phase difference between the operating voltage and the operating current on the ultrasonic atomization sheet 12 is small, and even the same phase between the operating voltage and the operating current can be maintained. Then, a heating power of the ultrasonic atomization sheet 12 can be reduced, that is, a heating temperature of the ultrasonic atomization sheet 12 is reduced. In addition, a useful power of the ultrasonic atomization sheet 12 is also increased, to improve operating efficiency of the ultrasonic atomization sheet 12 as a whole.

In addition, for N ultrasonic atomization apparatuses 100, one ultrasonic atomization sheet 12 is disposed in each ultrasonic atomization apparatus 100, and there are N ultrasonic atomization sheets 12 in total, where N is an integer greater than or equal to 1. Individual performance of different ultrasonic atomization sheets among the N ultrasonic atomization sheets 12 usually varies, even if the N ultrasonic atomization sheets 12 are ultrasonic atomization sheets in a same batch. In this case, the same capacitance branch 1331 and inductance branch 1332 are disposed in each of the N ultrasonic atomization apparatuses 100, so that a variation range of a capacitance value of an overall equivalent capacitance of N first circuits A1 in the N ultrasonic atomization apparatuses 100 can be controlled within a small range, and an overall impedance feature of N second circuits A2 in the N ultrasonic atomization apparatuses 100 can be switched to be inductive. In this case, heating temperatures of the N ultrasonic atomization sheets 12 are low. In addition, in this case, a same control parameter may be used for the N ultrasonic atomization apparatuses 100, so that control difficulty can be reduced, a yield rate in a production process can be increased, and overall production efficiency can be improved.

In addition, because the impedance feature of the second circuit A2 remains inductive, inductive load can impede a change in a current. Therefore, there is no large overshoot current in a start-up process of the ultrasonic atomization apparatus 100. For example, an operating voltage of the ultrasonic atomization sheet 12 in a start-up stage may be set to a maximum value of an operating voltage in an entire operating cycle, so that the ultrasonic atomization sheet 12 can be driven at a maximum voltage. In this way, in a start-up stage of the ultrasonic atomization sheet 12, there is no need to start up in a slowly manner, namely, a manner of gradually increasing a voltage in a start-up process. In this way, control difficulty can be reduced, and a first puff of vapor can be quickly generated.

Certainly, in some implementations, with reference to an actual application process of the ultrasonic atomization apparatus 100, the ultrasonic atomization sheet 12 enters a steady state after being started. In this case, due to heat generation of the ultrasonic atomization sheet 12, there is no need to maintain a large power to drive the ultrasonic atomization sheet 12. In other words, after entering the steady state, the driving voltage used to drive the ultrasonic atomization sheet 12 may be reduced. Specifically, in an embodiment, an operating voltage waveform of the ultrasonic atomization sheet 12 when the ultrasonic atomization sheet 12 operates may be an underdamped oscillation, or a decreasing oscillation whose oscillation amplitude gradually decreases.

In an embodiment, the capacitance branch 1331 is configured such that a capacitance value of the capacitance branch 1331 is less than a capacitance value of an equivalent capacitance of the ultrasonic atomization sheet 12 when the ultrasonic atomization sheet 12 operates at a series resonant frequency point.

In this embodiment, the capacitance value of the capacitance branch 1331 is configured to be less than the capacitance value of the equivalent capacitance of the ultrasonic atomization sheet 12 when the ultrasonic atomization sheet 12 operates at the series resonant frequency point, and because a total capacitance value of capacitors connected in series is necessarily less than the capacitance value of any series capacitor, no matter what ultrasonic atomization sheet 12 is selected, finally, the capacitance value of the equivalent capacitance of the first circuit A1 is necessarily a value less than the capacitance value of the capacitance branch 1331. For example, in an implementation, the capacitance value of the equivalent capacitance of the ultrasonic atomization sheet 12 when the ultrasonic atomization sheet 12 operates at the series resonant frequency point is 5.9 nF, and the capacitance value of the capacitance branch 1331 is configured to be 4.7 nF. In this case, the capacitance value of the equivalent capacitance of the first circuit A1 is, for example, less than 4.7 nF.

Then, a proper capacitance branch 1331 is selected, so that the capacitance value of the equivalent capacitance of the first circuit A1 can be configured to be a required value. That is, the capacitance value of the equivalent capacitance of the first circuit A1 is a controllable value. A user can perform corresponding configuration according to different application scenarios, and practicability is strong.

In this case, it may be determined that the capacitance value of the equivalent capacitance of the first circuit A1 is necessarily less than the capacitance value of the capacitance branch 1331. In an implementation, the capacitance value of the capacitance branch 1331 may be used as a maximum value of the equivalent capacitance of the first circuit A1, and a minimum value of the inductance value of the inductance branch 1332 may be calculated according to a condition of switching the impedance feature of the second circuit A2 to be inductive. Then, the inductance value of the inductance branch 1332 that is actually used is set to be greater than or equal to the minimum value. In this way, regardless of how the capacitance value of the equivalent capacitance of the ultrasonic atomization sheet 12 changes, the capacitance value of the equivalent capacitance of the first circuit A1 is always less than the capacitance value of the capacitance branch 1331, and the impedance feature of the second circuit A2 can always be maintained as inductive.

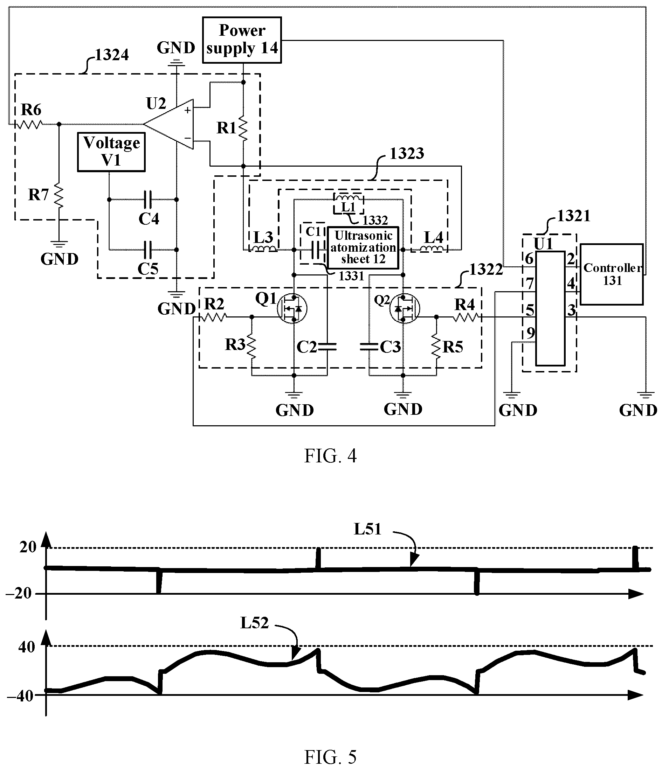

FIG. 4 exemplarily shows a structure of a capacitance branch 1331. As shown in FIG. 4, the capacitance branch 1331 includes a first capacitor C1, and the first capacitor C1 and an ultrasonic atomization sheet 12 are connected in series. The first capacitor C1 may be disposed on a left side of the ultrasonic atomization sheet 12, or may be disposed on a right side of the ultrasonic atomization sheet 12. This is not specifically limited in this embodiment of this application.

In an embodiment, a capacitance value of the first capacitor C1 is any value in [1 nF, 20 nF]. In this embodiment, usually, an ultrasonic atomization sheet 12 with a vibration frequency of 3 MHz should be selected. However, in other embodiments, the capacitance value of the first capacitor C1 may be correspondingly set according to an actual application condition. For example, in an implementation, if another vibration frequency (for example, 2.7 MHz) is selected, a selected value range of the first capacitor C1 should be correspondingly modified.

It should be noted that, in this embodiment, a value range of the first capacitance C1 may be obtained through testing of different ultrasonic atomization sheets 12, so that when the first capacitance C1 is used, even if a capacitance value of an equivalent capacitance of the ultrasonic atomization sheet 12 changes during operating or individual performance of the ultrasonic atomization sheet 12 varies, safety and reliability of the ultrasonic atomization sheet 12 during use can still be ensured.

In an implementation, a specific implementation process includes: testing to-be-used ultrasonic atomization sheets 12 in a same batch, to obtain an equivalent capacitance of each ultrasonic atomization sheet 12 when each ultrasonic atomization sheet 12 operates at a series resonant point, and determine a minimum value among the equivalent capacitances. Then, to reduce a capacitance value of load during actual operating, the capacitance value of the first capacitor C1 may be selected to be less than a minimum value, to limit the capacitance value of the overall equivalent capacitance of the first circuit A1 to be near the capacitance value of the first capacitor C1. In this way, even if the capacitance value of the equivalent capacitance of the ultrasonic atomization sheet 12 continuously changes during operating, or individual performance of the ultrasonic atomization sheet 12 varies, the capacitance value of the equivalent capacitance of the first circuit A1 is always maintained near the capacitance value of the first capacitor C1, and a change range is small, which helps to more simply set the corresponding inductance branch 1332 to maintain the impedance feature of the second circuit A2 as inductive.

In addition, it may be understood that, the capacitance value of the first capacitor C1 cannot be excessively small. Otherwise, impedance of the first capacitor C1 is excessively large, resulting in a small current flowing through the ultrasonic atomization sheet 12. In this case, a useful power on the ultrasonic atomization sheet 12 is also low. Certainly, in other embodiments, the capacitance value of the first capacitor C1 may alternatively be selected to be greater than or equal to a minimum value of the foregoing equivalent capacitances. Although the capacitance value of the equivalent capacitance of the first circuit A1 can be kept fluctuating within a small range, the capacitance value of the equivalent capacitance of the first circuit A1 is greatly affected by parameters of the ultrasonic atomization sheet 12. Therefore, when the capacitance value of the equivalent capacitance of the ultrasonic atomization sheet 12 is excessively large, there is still a risk that the impedance feature of the second circuit A2 is switched to be capacitive.

In summary, the capacitance value of the first capacitor C1 is set to any value in [1 nF, 20 nF]. An abnormal phenomenon in which a current flowing through the ultrasonic atomization sheet 12 is small because the first capacitor C1 is set to be excessively small can be prevented. In addition, an abnormal phenomenon such as severe heating or inconsistent atomization performance of the ultrasonic atomization sheet 12 caused by changes in the parameters of the ultrasonic atomization sheet 12 during operating or different individual performance of different ultrasonic atomization sheets 12 can be effectively prevented. Therefore, for the ultrasonic atomization sheet 12 with the vibration frequency of 3 MHz, the capacitance value range [1 nF, 20 nF] of the first capacitor C1 is a proper range, which not only reduces a heating temperature of the ultrasonic atomization sheet 12, but also is applicable to different ultrasonic atomization sheets 12 with the vibration frequency of 3 MHz.

FIG. 4 further exemplarily shows a structure of an inductance branch 1332. As shown in FIG. 4, the inductance branch 1332 includes a first inductor L1. The first inductor L1 and a first circuit A1 (namely, a circuit formed by a first capacitor C1 and an ultrasonic atomization sheet 12 that are connected in series) are connected in parallel.

In an embodiment, an inductance value of the first inductor L1 is any value in [0.1 µH, 2 µH]. In this embodiment, an example in which the ultrasonic atomization sheet 12 with a vibration frequency of 3 MHz is selected is used. However, in other embodiments, the inductance value of the first inductor L1 may be correspondingly set according to an actual application condition (for example, a vibration frequency of the used ultrasonic atomization sheet 12).

In this embodiment, a lower limit of the inductance value of the first inductor L1 is set to 0.1 µH, so that it can be ensured that the first inductor L1 can switch the overall impedance feature of the second circuit A2 to be inductive. In this way, a phase between the operating voltage and the operating current that are applied to the ultrasonic atomization sheet 12 is within a relatively small range, to improve efficiency of the power supply 14 and reduce a heating power on the ultrasonic atomization sheet 12, so as to reduce a heating temperature of the ultrasonic atomization sheet 12. In addition, an upper limit of the inductance value of the first inductor L1 is set to 2 µH, so that an abnormal phenomenon in which an alternating current is excessively small and a useful power obtained on the ultrasonic atomization sheet 12 is excessively small due to an excessively large hindering force of the second circuit A2 on the alternating current can be prevented, to maintain the efficiency of the power supply 14.

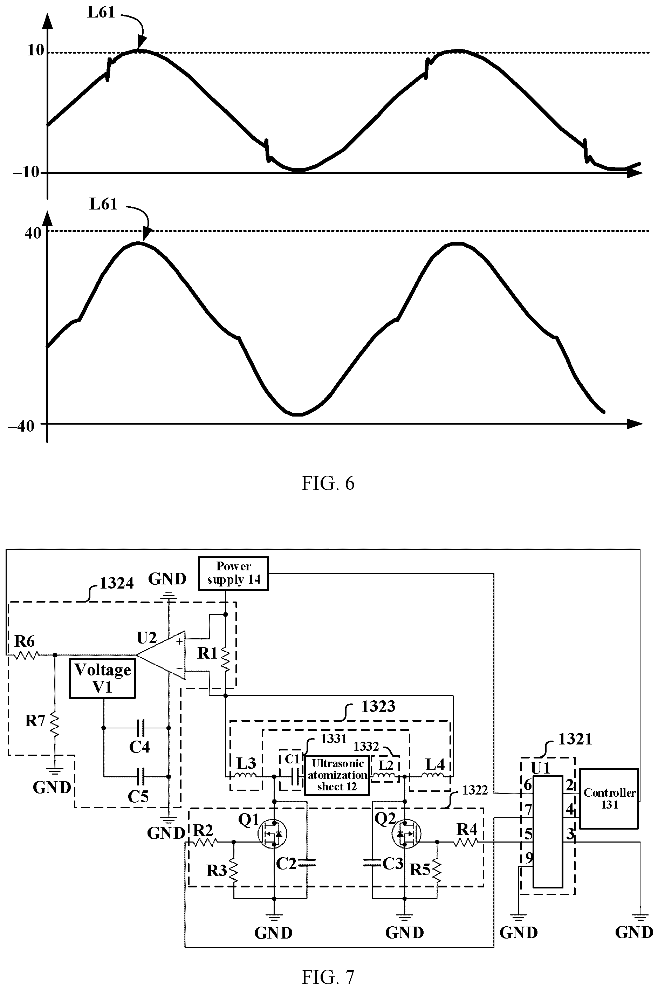

Referring to both FIG. 5 and FIG. 6, FIG. 5 shows an operating voltage and an operating current that are applied to an ultrasonic atomization sheet 12 when a capacitance branch 1331 and an inductance branch 1332 are not added, where a curve L51 is the operating current applied to the ultrasonic atomization sheet 12; and a curve L52 is an operating voltage applied to the ultrasonic atomization sheet 12. FIG. 6 shows an operating voltage and an operating current that are applied to an ultrasonic atomization sheet 12 when a capacitance branch 1331 and an inductance branch 1332 are added, where a curve L61 is the operating current applied to the ultrasonic atomization sheet 12; and a curve L62 is an operating voltage applied to the ultrasonic atomization sheet 12.

In this embodiment, when the capacitance branch 1331 and the inductance branch 1332 are not added, it can be learned from the curve L51 that, the operating current applied to the ultrasonic atomization sheet 12 is severely distorted and easily changes abruptly, and the current is large (about 20 mA) when the operating current changes abruptly, which is likely to cause the power supply to burn out. In addition, it can be learned with reference to the curve L51 and the curve L52 that, a phase difference between the operating voltage and the operating current that are applied to the ultrasonic atomization sheet 12 is relatively large, and a useful power applied to the ultrasonic atomization sheet 12 is relatively low. Most of the power provided by the power supply 14 is converted into a heating power of the ultrasonic atomization sheet 12, resulting in severe heating of the ultrasonic atomization sheet 12, and even possible damage to components such as the ultrasonic atomization sheet 12.

When the capacitance branch 1331 and the inductance branch 1332 are added, it can be learned from the curve L61 that, a distortion of the operating current applied to the ultrasonic atomization sheet 12 is reduced, and a current is small (where the current is always kept between [-10 mA, 10 mA]) when the operating current changes abruptly, causing less damage to the power supply. In addition, it may be correspondingly determined that a variation amplitude of an output power supply of the power supply 14 is also small. In this case, when a driving power supply is controlled by detecting a variation of the output power supply of the power supply 14 (for example, tracking a series resonant frequency point), control is stable, simple, safe, and reliable, and there is no large overshoot current, so that control complexity can be greatly reduced.

In addition, it can be learned from the curve L61 and the curve L62 that, a phase difference between the operating voltage and the operating current that are applied to the ultrasonic atomization sheet 12 is small, or the operating voltage and the operating current may even maintain the same phase. For example, in an implementation, when the ultrasonic atomization sheet operates, the phase difference between the operating current and the operating voltage that are applied to the ultrasonic atomization sheet 12 is less than 40°. In this way, a useful power applied to the ultrasonic atomization sheet 12 is high. Most of the power provided by the power supply 14 is converted into an operating power of the ultrasonic atomization sheet 12, so that a heating power of the ultrasonic atomization sheet 12 can be reduced to reduce a heating temperature of the ultrasonic atomization sheet 12, thereby improving operating efficiency and stability of the ultrasonic atomization sheet 12.

FIG. 7 further exemplarily shows another structure of an inductance branch 1332. As shown in FIG. 7, the inductance branch 1332 includes a second inductor L2.

The second inductor L2, a capacitance branch 1331, and the ultrasonic atomization sheet 12 are connected in series. Specifically, the second inductor L2 and a first circuit A1 are connected in series, and the second inductor L2 is disposed on a left side or a right side of the first circuit A1; or the second inductor L2 is connected between the capacitance branch 1331 and the ultrasonic atomization sheet 12.

In an implementation, an inductance value of the second inductor L2 is any value in [1 µH, 4.7 µH].

In this embodiment, an application condition of the second inductor L2 is similar to that of a first inductor L1, which is within a scope easily understood by a person skilled in the art, and details are not described herein again.

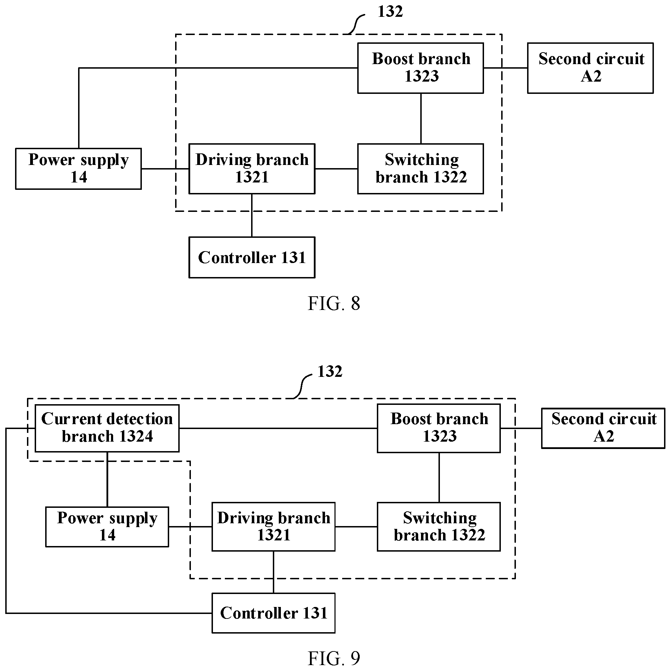

In an embodiment, as shown in FIG. 8, a driving circuit 132 includes a driving branch 1321, a switching branch 1322, and a boost branch 1323. The driving branch 1321 is separately connected to a controller 131 and a power supply 14, the switching branch 1322 is connected to the driving branch 1321, and the boost branch 1323 is separately connected to the power supply 14, the switching branch 1322, and a second circuit A2.

Specifically, the driving branch 1321 is configured to output a second pulse signal in response to a first pulse signal output by the controller 131 and a current output by the power supply 14. The switching branch 1322 is configured to be turned on or turned off in response to the second pulse signal. The boost branch 1323 is configured to boost an output voltage of the power supply 14 in response to turn-on or turn-off of the switching branch 1322, to generate a first driving signal used to drive the ultrasonic atomization sheet 12.

FIG. 4 exemplarily shows a structure of a switching branch 1322. As shown in FIG. 4, the switching branch 1322 includes a first switch Q1 and a second switch Q2. The first switch Q1 is separately connected to the driving branch 1321 and the boost branch 1323, and the second switch Q2 is separately connected to the driving branch 1321 and the boost branch 1323.

Specifically, in an embodiment, both a first end of the first switch Q1 and a first end of the second switch Q2 are connected to the driving branch 1321, both a second end of the first switch Q1 and a second end of the second switch Q2 are connected to ground GND, a third end of the first switch Q1 is separately connected to a first end of the boost branch 1323 and a first end of the second circuit A2, and a third end of the second switch Q2 is separately connected to a second end of the boost branch 1323 and a second end of the second circuit A2.

If the second pulse signal output by the driving branch 1321 includes a first pulse sub-signal and a second pulse sub-signal, the first switch Q1 is configured to be turned on or turned off in response to the first pulse sub-signal, to generate a first voltage signal, and the second switch Q2 is configured to be turned on or turned off in response to the second pulse sub-signal, to generate a second voltage signal. A driving voltage used to drive the ultrasonic atomization sheet 12 includes a first voltage signal and a second voltage signal.

In addition, the first switch Q 1 and the second switch Q2 are alternately turned on. In other words, when the first switch Q1 is turned on, the second switch Q2 is turned off. When the first switch Q1 is turned off, the second switch Q2 is turned on. In an implementation, both the first switch Q1 and the second switch Q2 are turned on and turned off at a duty cycle of 50%.

In this embodiment, when the first pulse signal output by the driving branch 1321 includes two sub-signals, the first pulse signal output by the controller 131 should also correspondingly include two sub-signals, which are respectively a third pulse sub-signal and a fourth pulse sub-signal. When the controller 131 outputs the third pulse sub-signal, the driving branch 1321 outputs the first pulse sub-signal. When the controller 131 outputs the fourth pulse sub-signal, the driving branch 1321 outputs the second pulse sub-signal.

It should be noted that, in this embodiment, an example in which both the first switch Q1 and the second switch Q2 are N-type metal-oxide-semiconductor field-effect transistors (referred to as NMOS transistors for short below) is used.

A gate of the NMOS transistor is a first end of the first switch Q1, a source of the NMOS transistor is a second end of the first switch Q1, and a drain of the NMOS transistor is a third end of the first switch Q1. The gate of the NMOS transistor is a first end of the second switch Q2, the source of the NMOS transistor is a second end of the second switch Q2, and the drain of the NMOS transistor is a third end of the second switch Q2.

In addition, in other embodiments, the first switch Q1 and the second switch Q2 may alternatively be P-type metal-oxide-semiconductor field-effect transistors or signal relays, and the first switch Q1 and the second switch Q2 may alternatively be at least one of triodes, insulated gate bipolar transistors, integrated gate commutated thyristors, gate turn-off thyristors, junction gate field-effect transistors, MOS controlled thyristors, gallium nitride-based power devices, silicon carbide-based power devices, or thyristors.

In an embodiment, the switching branch 1322 further includes a first switching branch capacitor C2 and a second switching branch capacitor C3. A first end of the first switching branch capacitor C2 is connected to a second end of the first switch Q1, a second end of the first switching branch capacitor C2 is connected to a third end of the first switch Q1, a first end of the second switching branch capacitor C3 is connected to a second end of the second switch Q2, and a second end of the second switching branch capacitor C3 is connected to a third end of the second switch Q2.

In this embodiment, the first switching branch capacitor C2 and the second switching branch capacitor C3 are zero-crossing adjustment capacitors of the first switch Q1 and the second switch Q2 respectively. The first switching branch capacitor C2 and the second switching branch capacitor C3 are added, so that transient currents and transient voltages on the first switch Q1, the second switch Q2, and the ultrasonic atomization sheet 12 are facilitated, even if the voltages change slowly, to prevent a large impact on components, thereby improving the operating efficiency of the power supply 14.

In an embodiment, the switching branch 1322 further includes a second resistor R2, a third resistor R3, a fourth resistor R4, and a fifth resistor R5. A first end of the second resistor R2 is connected to a seventh pin of a driver chip U1, a second end of the second resistor R2 is separately connected to a first end of the third resistor R3 and a first end of the first switch Q1, both a second end of the third resistor R3 and a second end of the first switch Q1 are connected to the ground GND, and a third end of the first switch Q1 is connected to the boost branch 1323 and the second circuit A2. A first end of the fourth resistor R4 is connected to a fifth pin of the driver chip U1, a second end of the fourth resistor R4 is separately connected to a first end of the fifth resistor R5 and a first end of the second switch Q2, both a second end of the fifth resistor R5 and a second end of the second switch Q2 are connected to the ground GND, and a third end of the second switch Q2 is connected to the boost branch 1323 and the second circuit A2.

In this embodiment, the second resistor R2 and the third resistor R3 are configured to divide a voltage of a pulse signal output by the seventh pin of the driver chip U1, to obtain a voltage of the first end of the first switch Q1. When a divided voltage of the third resistor R3 is greater than a turn-on voltage of the first switch Q1, the first switch Q1 is turned on, or otherwise the first switch Q1 is turned off.

The fourth resistor R4 and the fifth resistor R5 are configured to divide a voltage of a pulse signal output by the fifth pin of the driver chip U1, to obtain a voltage of the first end of the second switch Q2. When a divided voltage of the fifth resistor R5 is greater than a turn-on voltage of the second switch Q2, the second switch Q2 is turned on, or otherwise the second switch Q2 is turned off.

FIG. 4 further exemplarily shows a structure of the driving branch 1321. As shown in FIG. 4, the driving branch 1321 includes a driver chip U1, and the driver chip U1 includes a power supply input end, a first signal input end, a second signal input end, a first signal output end, and a second signal output end. In this embodiment, the power supply input end is a sixth pin of the driver chip U1, the first signal input end is a second pin of the driver chip U1, the second signal input end is a fourth pin of the driver chip U1, the first signal output end is a fifth pin of the driver chip U1, and the second signal output end is a seventh pin of the driver chip U1.

Specifically, a sixth pin of the driver chip U1 is configured to be connected to the power supply 14. Both the second pin and the fourth pin of the driver chip U1 are connected to the controller 131. Both the fifth pin and the seventh pin of the driver chip U1 are connected to the switching branch 1322. The second pin and the fourth pin of the driver chip U1 are configured to input a first pulse signal, and the fifth pin and the seventh pin of the driver chip U1 are configured to output a second pulse signal. The third pulse sub-signal is input into the second pin of the driver chip U1, the fourth pulse sub-signal is input into the fourth pin of the driver chip U1, the seventh pin of the driver chip U1 outputs the first pulse sub-signal, and the fifth pin of the driver chip U1 outputs the second pulse sub-signal.

In this embodiment, the driver chip U1 is disposed, to improve a driving capability of the pulse signal output by the controller 131. Therefore, the switching branch 1322 can be driven quickly, to maintain stable operation of the ultrasonic atomization sheet 12. In addition, a larger current input into the sixth pin of the driver chip U1 indicates a stronger driving capability output by the fifth pin and the seventh pin of the driver chip U1.

In an embodiment, the driver chip U1 may be an integrated chip whose model is SGM48000. Certainly, in other embodiments, an integrated chip of another model may alternatively be used. This is not limited in this embodiment of this application. In addition, because there are different types of driver chips, when a driver chip of another type is used, specific definitions of a pin may be different, but functions and signal definitions of the pins are the same. If the driver chip of another type is used, the driver chip may be set in a manner similar to that in the foregoing embodiment. This is within a scope easily understood by a person skilled in the art, and details are not described herein again.

In addition, in this embodiment, an example in which the power supply 14 is used as an input power supply of the driver chip U1 is used. In other words, in this embodiment, the power supply 14 is used as a power supply of both the driver chip U1 and the ultrasonic atomization sheet 12, to reduce costs. However, in other embodiments, to prevent the driver chip U1 and the ultrasonic atomization sheet 12 from interfering with each other during operating, two different power supplies may be used to supply power to the driver chip U1 and the ultrasonic atomization sheet 12 respectively, to improve operating stability of the driver chip U1 and the ultrasonic atomization sheet 12.

FIG. 4 further exemplarily shows a structure of a boost branch 1323. As shown in FIG. 4, the boost branch 1323 includes a first boost branch inductor L3 and a second boost branch inductor L4. The first boost branch inductor L3 is separately connected to a third end of the first switch Q1, the power supply 14, and the second circuit A2, and the second boost branch inductor L4 is separately connected to a third end of the second switch Q2, the power supply 14, and the second circuit A2.

Specifically, the first boost branch inductor L3 is configured to be charged when the first switch Q1 is turned on, and generate, when the first switch Q1 is turned off, the first voltage signal used to drive the ultrasonic atomization sheet 12 according to a voltage of the power supply 14 and a charged voltage of the first boost branch inductor L3.

The second boost branch inductor L4 is configured to be charged when the second switch Q2 is turned on, and generate, when the second switch Q2 is turned off, the second voltage signal used to drive the ultrasonic atomization sheet 12 according to the voltage of the power supply 14 and a charged voltage of the second boost branch inductor L4.

In this embodiment, when the first switch Q1 is turned on and the second switch Q2 is turned off, the power supply 14, the first boost branch inductor L3, and the first switch Q1 form a loop, and the first boost branch inductor L3 is charged by the power supply 14. In addition, the power supply 14, the second boost branch inductor L4, the ultrasonic atomization sheet 12, and the first switch Q1 form a loop, and voltages on the power supply 14 and the second boost branch inductor L4 simultaneously provide a driving voltage for the ultrasonic atomization sheet 12.

When the second switch Q2 is turned on and the first switch Q1 is turned off, the power supply 14, the second boost branch inductor L4, and the second switch Q2 form a loop, and the second boost branch inductor L4 is charged by the power supply 14. In addition, the power supply 14, the first boost branch inductor L3, the ultrasonic atomization sheet 12, and the second switch Q2 form a loop, and voltages on the power supply 14 and the first boost branch inductor L3 simultaneously provide a driving voltage for the ultrasonic atomization sheet 12.

In an embodiment, as shown in FIG. 9, the driving circuit 132 further includes a current detection sub-branch 1324. The current detection sub-branch 1324 is separately connected to the power supply 14, the boost branch 1323, and the controller 131. Specifically, the current detection sub-branch 1324 is configured to detect a current flowing into the boost branch 1323.

In this embodiment, the controller 131 may obtain the current flowing into the boost branch 1323 through the current detection sub-branch 1324. Then, the controller 131 may determine, according to the current, whether the ultrasonic atomization sheet 12 has an abnormality such as an excessively large current during operating, so that processing can be performed in time when the abnormality occurs, to help reduce a risk of damage to the ultrasonic atomization sheet 12.

FIG. 4 further exemplarily shows a structure of the current detection sub-branch 1324. As shown in FIG. 4, the current detection sub-branch 1324 includes an amplifier U2 and a first resistor R1. The first resistor R1 is separately connected to the amplifier U2 and the boost branch 1323, and the amplifier U2 is connected to the controller 131.

Specifically, a first end of the first resistor R1 is separately connected to the power supply 14 and a non-inverting input end of the amplifier U2, a second end of the first resistor R1 is separately connected to an inverting input end of the amplifier U2, a first end of the first boost branch inductor L3, and a first end of the second boost branch inductor L4, an output end of the amplifier U2 is connected to the controller 13, a ground end of the amplifier U2 is connected to the ground GND, and a power supply end of the amplifier U2 is connected to a voltage V1.

In this embodiment, the amplifier U2 is configured to output a detection voltage according to voltages on two ends of the first resistor R1, so that the controller 131 determines a current flowing into the boost branch 1323 based on the detection voltage. Specifically, the amplifier U2 can amplify a received voltage on two ends of the first resistor R1 by K times to output the detection voltage, where K is a positive integer. Then, after obtaining the detection voltage, the controller 131 may determine the current flowing into the boost branch 1323 according to a relationship between the detection voltage and the current flowing into the boost branch 1323.

In an embodiment, the current detection sub-branch 1324 further includes a fourth capacitor C4, a fifth capacitor C5, a sixth resistor R6, and a seventh resistor R7. The fourth capacitor C4 and the fifth capacitor C5 are filter capacitors, the sixth resistor R6 is a current limiting resistor, and the seventh resistor R7 is a pull-down resistor.

It should be noted that, in the embodiments shown in the foregoing figures, a representation form of the resistor is a single resistor, and a representation form of the capacitor is a single capacitor. In other embodiments, the resistors may alternatively be integrated with resistors connected in series, resistors connected in parallel, or resistors connected in a hybrid manner, and the capacitors may alternatively be integrated with capacitors connected in series, capacitors connected in parallel, or capacitors connected in a hybrid manner.

A connection described in this application may be a direct connection, namely, a connection between two components, or may be an indirect connection, that is, an indirect connection may be formed between two components through one or more elements.

Finally, it should be noted that the foregoing embodiments are merely used for describing the technical solutions of this application, but are not intended to limit the present utility model. Under the concept of this application, the technical features in the foregoing embodiments or different embodiments may be combined, the steps may be implemented in any sequence, and there may be many other changes in different aspects of this application as described above. For brevity, those are not provided in detail. Although this application is described in detail with reference to the foregoing embodiments, a person of ordinary skill in the art should understand that they may still make modifications to the technical solutions described in the foregoing embodiments or make equivalent replacements to some technical features thereof, without departing from the scope of the technical solutions of the embodiments of this application.

## Claims

1. An ultrasonic atomization apparatus, comprising:
a liquid storage cavity, configured to store a liquid substrate;
an ultrasonic atomization sheet, wherein the ultrasonic atomization sheet is in liquid communication with the liquid storage cavity, and the ultrasonic atomization sheet is configured to generate oscillation to atomize the liquid substrate; and
a power supply and a driving control circuit, wherein
the driving control circuit comprises a controller, a driving circuit, and an impedance matching circuit, wherein
the impedance matching circuit comprises a capacitance branch and an inductance branch; the capacitance branch and the ultrasonic atomization sheet are connected in series to form a first circuit, and the inductance branch is connected to the first circuit to form a second circuit; and
the driving circuit is separately connected to the controller, the power supply, and the second circuit, and the driving circuit is configured to output, based on control of the controller, a driving voltage used to drive the ultrasonic atomization sheet.

2. The ultrasonic atomization apparatus according to claim 1, wherein the capacitance branch is configured such that a capacitance value of the capacitance branch is less than a capacitance value of an equivalent capacitance of the ultrasonic atomization sheet when the ultrasonic atomization sheet operates at a series resonant frequency point.

3. The ultrasonic atomization apparatus according to claim 1 or 2, wherein the capacitance branch comprises a first capacitor.

4. The ultrasonic atomization apparatus according to claim 3, wherein a capacitance value of the first capacitor is any value in [1 nF, 20 nF].

5. The ultrasonic atomization apparatus according to claim 1 or 2, wherein the inductance branch comprises a first inductor; and
the first inductor and the first circuit are connected in parallel.

6. The ultrasonic atomization apparatus according to claim 5, wherein an inductance value of the first inductor is any value in [0.1 µH, 2 µH].

7. The ultrasonic atomization apparatus according to claim 1 or 2, wherein the inductance branch comprises a second inductor; and
the second inductor, the capacitance branch, and the ultrasonic atomization sheet are connected in series.

8. The ultrasonic atomization apparatus according to claim 7, wherein an inductance value of the second inductor is any value in [1 µH, 4.7 µH].

9. The ultrasonic atomization apparatus according to claim 1, wherein the driving circuit comprises a driving branch, a switching branch, and a boost branch, wherein
the driving branch is separately connected to the controller and the power supply, and the driving branch is configured to output a second pulse signal in response to a first pulse signal output by the controller and a current output by the power supply;
the switching branch is connected to the driving branch, and the switching branch is configured to be turned on or turned off in response to the second pulse signal; and
the boost branch is separately connected to the power supply, the switching branch, and the second circuit, and the boost branch is configured to boost an output voltage of the power supply in response to turn-on or turn-off of the switching branch, to generate the driving voltage.

10. The ultrasonic atomization apparatus according to claim 9, wherein the switching branch comprises a first switch and a second switch, the first switch is separately connected to the driving branch and the boost branch, and the second switch is separately connected to the driving branch and the boost branch; and
the first switch and the second switch are configured to be alternately turned on.

11. The ultrasonic atomization apparatus according to claim 10, wherein the switching branch further comprises a first switching branch capacitor and a second switching branch capacitor; and
a first end of the first switching branch capacitor is connected to a second end of the first switch, a second end of the first switching branch capacitor is connected to a third end of the first switch, a first end of the second switching branch capacitor is connected to a second end of the second switch, and a second end of the second switching branch capacitor is connected to a third end of the second switch.

12. The ultrasonic atomization apparatus according to claim 10, wherein the boost branch comprises a first boost branch inductor and a second boost branch inductor; and
the first boost branch inductor is separately connected to the first switch, the power supply, and the second circuit, and the second boost branch inductor is separately connected to the second switch, the power supply, and the second circuit.

13. The ultrasonic atomization apparatus according to claim 1 or 2, wherein when the ultrasonic atomization sheet operates, an operating current and an operating voltage that are applied to the ultrasonic atomization sheet maintain a same phase; or
when the ultrasonic atomization sheet operates, a phase difference between an operating current and an operating voltage that are applied to the ultrasonic atomization sheet is less than 40°.

14. The ultrasonic atomization apparatus according to claim 1 or 2, wherein an operating voltage waveform of the ultrasonic atomization sheet when the ultrasonic atomization sheet operates is an underdamped oscillation or a decreasing oscillation.

15. The ultrasonic atomization apparatus according to claim 1 or 2, wherein the operating voltage of the ultrasonic atomization sheet at a start-up stage is a maximum value of the operating voltage in an entire operating cycle.

16. An ultrasonic atomization apparatus, comprising:
a liquid storage cavity, configured to store a liquid substrate;
an ultrasonic atomization sheet, wherein the ultrasonic atomization sheet is in liquid communication with the liquid storage cavity, and the ultrasonic atomization sheet is configured to generate oscillation to atomize the liquid substrate; and
a power supply and a driving control circuit, wherein
the driving control circuit comprises a controller, a dual-switch boost driving circuit, and an impedance matching circuit, wherein
the impedance matching circuit comprises a capacitance branch and an inductance branch; the capacitance branch and the ultrasonic atomization sheet are connected in series to form a first circuit, and the inductance branch is connected to the first circuit to form a second circuit; and
the dual-switch boost driving circuit is separately connected to the controller, the power supply, and the second circuit, and the dual-switch boost driving circuit is configured to alternately output, based on control of the controller, a first driving voltage and a second driving voltage that are used to drive the ultrasonic atomization sheet.

17. The ultrasonic atomization apparatus according to claim 16, wherein the capacitance branch comprises a first capacitor, and a capacitance value of the first capacitor is less than a capacitance value of an equivalent capacitance of the ultrasonic atomization sheet when the ultrasonic atomization sheet operates at a series resonant frequency point.

18. The ultrasonic atomization apparatus according to claim 16 or 17, wherein the inductance branch comprises a first inductor connected in parallel with the first circuit, or the inductance branch comprises a second inductor connected in series with the ultrasonic atomization sheet.

19. An ultrasonic atomization apparatus, comprising:
a power supply mechanism and an ultrasonic atomizer, wherein
the ultrasonic atomizer comprises:
a first housing, wherein a liquid storage cavity is formed in the first housing and is configured to store a liquid substrate; and
an ultrasonic atomization sheet disposed in the first housing, wherein the ultrasonic atomization sheet is in liquid communication with the liquid storage cavity, and the ultrasonic atomization sheet is configured to generate oscillation when receiving a driving voltage, to atomize the liquid substrate; and
the power supply mechanism comprises:
a second housing; and
a power supply and a driving control circuit that are disposed in the second housing, wherein the driving control circuit comprises a controller, a driving circuit, and an impedance matching circuit, the driving circuit is separately connected to the power supply and the controller, and the impedance matching circuit comprises a capacitance branch and an inductance branch;
when the first housing is coupled to the second housing, the capacitance branch and the ultrasonic atomization sheet are connected in series to form a first circuit, the inductance branch is connected to the first circuit to form a second circuit, and the driving circuit is connected to the second circuit; and the driving circuit is configured to output, based on control of the controller, a driving voltage used to drive the ultrasonic atomization sheet.

20. The ultrasonic atomization apparatus according to claim 19, wherein the capacitance branch comprises a first capacitor, and a capacitance value of the first capacitor is less than a capacitance value of an equivalent capacitance of the ultrasonic atomization sheet when the ultrasonic atomization sheet operates at a series resonant frequency point.

21. The ultrasonic atomization apparatus according to claim 19 or 20, wherein the inductance branch comprises a first inductor connected in parallel with the first circuit, or the inductance branch comprises a second inductor connected in series with the ultrasonic atomization sheet.
